# EUROPEAN PATENT SPECIFICATION

(11) **EP 2 780 047 B1**
(45) Date of publication and mention of the grant of the patent: **19.12.2018**
(21) Application number: 12808439.9
(22) Date of filing: 13.11.2012
(51) Int. Cl.: A61L 27/18, A61L 27/44

(54) **A PROSTHESIS**
PROTHESE
PROTHÈSE

(30) Priority: 17.11.2011 GB 201119810
(43) Date of publication of application: 24.09.2014
(73) Proprietor: Biomet UK Healthcare Limited, Bridgend South Wales CF31 3XA (GB)
(72) Inventor: BIGSBY, Robert John Andrew, Penarth Glamorgan CF64 1EJ (GB); KHAN, Mohammed Imran, Reading Berkshire RG1 3PP (GB)
(74) Representative: Mays, Julie
(86) International application number: PCT/GB2012/052819
(87) International publication number: WO 2013/072679

(56) References cited:
- EP-A1- 1 923 079
- GB-A- 2 463 066
- US-A1- 2004 122 524
- US-A1- 2006 184 251
- US-A1- 2007 073 410
- US-A1- 2008 109 081
- US-A1- 2009 164 023

## Description

The present invention relates to a hip resurfacing prosthesis.

### Background

It is known to replace some or all of a natural bone joint that has become damaged or diseased with prosthetic components. For example, a natural hip joint that has become degraded may be replaced with an artificial joint comprising a prosthetic acetabular cup component which is implanted into the patient's acetabulum, and a prosthetic femoral head component which is implanted into the patient's femur and which articulates with the acetabular cup. A prosthetic joint of this type is known as a total hip replacement. Alternatively, a hip resurfacing procedure may be employed in which a cap is placed over the existing head of the femur. The cap articulates with an acetabular cup in much the same way as in a total hip replacement. However, as the existing femoral head is conserved in a resurfacing procedure, very little bone is removed in comparison to a total hip replacement.

US2006/184251 A1 discloses a medical device including a structural member having a surface wherein at least a portion of the surface is coated with a nanostructured material to a thickness of at least about 25 micrometers, and wherein the nanostructured material comprises a ceramic, ceramic composite, ceramic metal composite, or a combination thereof. The first structural member can be a femoral head and the second structural member can be an acetabular socket. The coating is disposed on the first surface. The first and second structural members can be made of fiber-reinforced polymers.

The conventional means of fixation of the acetabular cup is to impact the cup into the prepared acetabulum, with the additional use of bone cement if required. This is acceptable in many cases but does not offer alternatives for patients with a high level of degradation of the acetabular bone tissue. Additional fixation may be provided by bone screws which pass through the cup and engage deeply into the patient's bone tissue to hold the cup in place. Typically, a liner is then fitted in to the cup, over the screw heads to provide the articulating surface for the femoral head. GB0815884.2 discloses a monoblock cup formed from carbon fibre reinforced polyether ether ketone (CFR-PEEK) that comprises openings through which screws or the like (also formed from CFR-PEEK) may pass to attach the cup to the acetabulum. CFR-PEEK operates under boundary lubrication and consequently the presence of openings does not affect the wear performance of the bearing surface.

However, there have been studies which suggest that the carbon fibres in the CFR-PEEK material may scratch the surface of the femoral head component. This may result in the accelerated abrasive wear of the acetabular cup. It is therefore desirable to form the femoral head component from a ceramic material which is resistant to scratching from the carbon fibres. Nevertheless, to make the femoral head component in ceramic is more technically demanding than a metal component. Furthermore, for resurfacing procedures, it would be necessary for a ceramic resurfacing component to be thicker than a corresponding metal component. Consequently, a ceramic S component would potentially require more bone removal than would be necessary with a metal resurfacing component.

The present invention seeks to address some or all of the above mentioned issues.

### Statements of Invention

In accordance with an aspect of the invention there is provided a prosthesis comprising: a first component comprising an acetabular cup formed from a fibre reinforced polymer material and having a bearing surface; and a second component comprising a metal femoral head resurfacing component including a spherical cap portion and a central post extending from an inner surface of the spherical cap portion, wherein the inner surface of the spherical cap portion and the post have a rough surface, the second component being adapted to articulate with the bearing surface of the first component, wherein a surface treatment is applied to at least a portion of the bearing surface of the second component to form a scratch or wear-resistant surface, the surface treatment comprising a wear-resistant superlattice coating comprising a ceramic material.

The wear-resistant coating may prevent the bearing surface of the second component from being scratched by the fibres in the fibre reinforced polymer material. This may otherwise lead to the accelerated abrasive wear of the first component. Accordingly, the wear-resistant coating may improve the durability of the prosthesis.
The first component may be formed from carbon fibre reinforced polyether ether ketone (CFR-PEEK).

The surface treatment may comprise a hardening treatment, such as a diffusion hardening treatment.

The second component may be formed from a cobalt chrome alloy.

The wear-resistant coating may seal the metal of the second component from the joint interface and thus prevent metal ion release.

The acetabular cup may be a monoblock (i.e. one piece) cup.

The wear-resistant coating does not add significantly to the thickness of the second component, and thus may allow the thickness of the second component to be kept to a minimum. Consequently, the amount of bone which must be removed may be reduced.

The present invention may therefore be particularly suitable for resurfacing procedures.

The bearing surface of the first component may comprise one or more bores for receiving one or more attachment means to secure the first component in place.

The fibre reinforced polymer may operate under boundary lubrication and therefore the presence of the bores may not affect the wear performance of the bearing surface.

Furthermore, the bores may allow a surgeon to determine if the first component is correctly positioned.

### Brief Description of the Drawings

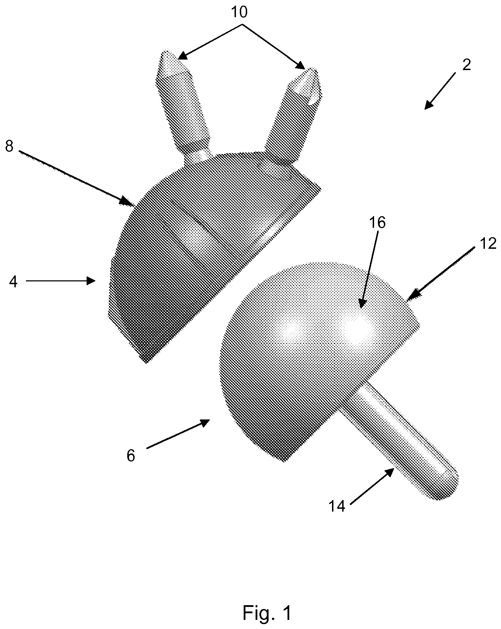

For a better understanding of the present disclosure, and to show more clearly how it may be carried into effect, reference will now be made, by way of example, to the accompanying drawing, in which:-
Figure 1 is an exploded side view of a prosthesis according to an embodiment of the invention.

### Detailed Description

With reference to Figure 1, a prosthesis 2 according to an embodiment of the invention comprises a acetabular cup 4 and a femoral resurfacing component 6.

The acetabular cup 4 is a monoblock component which is formed from a fibre reinforced polymer, such as carbon fibre reinforced polyether ether ketone (CFR-PEEK). The acetabular cup 4 is generally hemispherical and defines an inner surface (not shown) and an outer surface 8.

In use, the inner surface forms a bearing surface and the outer surface 8 contacts the suitably reamed acetabulum of the patient, as will be described in more detail below. The outer surface 8 of the acetabular cup 4 may be provided with a closed pore porous coating and/or a hydroxyapatite (HA) coating to aid fixation through osseointegration. Alternatively, the outer surface 8 may comprise a porous titanium bone in-growth surface having interconnected porosity. The outer surface 8 of the acetabular cup 4 may have a diameter of between 44 to 66mm. The diameter may be chosen to provide a precise anatomical fit with the acetabulum of the patient.

The acetabular cup 4 comprises a plurality of bores (not shown). The bores may extend through the acetabular cup 2 from the outer surface 8 to the inner surface. Alternatively, the bores may be blind bores which extend only partially into the acetabular cup 2 from the outer surface 8. The bores receive fixation means 10, such as screws, pins, barbs, spikes or any other suitable fixation device. The fixation means 10 may be used to enhance the fixation of the acetabular cup 2 with the acetabulum of the patient.

As described previously, CFR-PEEK operates under boundary lubrication and consequently the presence of the bores does not affect the wear performance of the bearing surface.

The femoral resurfacing component 6 is formed from a metallic material, such as a cobalt chrome alloy, and comprises a spherical cap portion 12 and a central post 14 extending from an inner surface of the spherical cap portion 12.

The inner surface of the spherical cap portion 12 and the post 14 may be grit blasted to provide a rough surface which aids integration of the femoral resurfacing component 6 with the patient's bone.

The spherical cap portion 12 may be between 38 to 60 mm in diameter and is selected to complement the inner bearing surface of the acetabular cup 4. The thickness of the spherical cap portion 12 is minimised in order to reduce the amount of bone removed from the femoral head.
The post 14 may be cylindrical and thus have a uniform cross-section along its length.

This may provide uniform stress transfer across the femoral head. Furthermore, the post 14 may be fluted to provide rotational stability.

An outer surface 16 of the spherical cap portion 12 forms a bearing surface and is provided with a wear-resistant coating. The wear-resistant coating is a superlattice coating comprising a ceramic material.

The acetabulum and femoral head of the patient is reamed prior to implantation of the prosthesis. The acetabular cup 4 is then impacted into the prepared acetabulum and the femoral resurfacing component 6 is cemented onto the prepared femoral head. The joint is then reduced so that the spherical cap portion 12 of the femoral resurfacing component 6 is received within the inner surface of the acetabular cup 4.

The wear-resistant coating on the outer surface 16 of the spherical cap portion 12 prevents the spherical cap portion 12 from being scratched by the carbon fibres in the CFR-PEEK material during the articulation of the acetabular cup 4 and femoral resurfacing component 6. Such scratching could otherwise lead to accelerated abrasive wear of the acetabular cup 4. Accordingly, the wear-resistant coating improves the durability of the prosthesis 2.
Further, the wear-resistant coating does not add significantly to the thickness of the spherical cap portion 12, thus allowing the thickness of the spherical cap portion 12 to be kept to a minimum. Consequently, the femoral resurfacing component 6 of the present invention reduces the amount of bone which must be removed from the femoral head when compared to a ceramic component.

Moreover, the wear-resistant coating seals the metal of the spherical cap portion 12 from the joint interface and thus prevents metal ion release (i.e. cobalt and chromium ions). Such metal ion release is undesirable since it may cause effects such as sensitivity reactions, pseudotumors, and genotoxicity.

The present invention therefore allows CFR-PEEK to be used to form the acetabular cup 4. This is desirable since, as described previously, CFR-PEEK allows additional fixation means to be used. This is not possible with a metal monoblock cup.

Furthermore, the bores through the acetabular cup 4 may allow the surgeon to determine whether the acetabular cup 4 is fully seated following impaction. The CFR-PEEK cup is also considered to be more forgiving than hard-on-hard bearings in situations where the cup is placed vertically.

Although the present invention has been described with reference to a hip resurfacing prosthesis, it may be applied to other types of prosthesis. For example, the invention may be applied to a total hip replacement prosthesis, or to prostheses for other anatomical joints, such as the knee or shoulder.

Other types of surface treatment may be performed on the outer surface 16 of the spherical cap portion 12 to form a scratch or wear-resistant surface. For example, the outer surface 16 may undergo a hardening treatment, such as diffusion hardening. The hardening treatment may be used in addition to the wear-resistant coating to form a duplex treatment.

## Claims

1. A prosthesis comprising:
a first component comprising an acetabular cup formed from a fibre reinforced polymer material and having a bearing surface; and
a second component comprising a metal femoral head resurfacing component including a spherical cap portion and a central post extending from an inner surface of the spherical cap portion, wherein the inner surface of the spherical cap portion and the post have a rough surface, the second component being adapted to articulate with the bearing surface of the first component, wherein a surface treatment is applied to at least a portion of the bearing surface of the second component to form a wear-resistant surface, the surface treatment comprising a wear-resistant superlattice coating comprising a ceramic material.

2. A prosthesis as claimed in claim 1, wherein the first component is formed from carbon fibre reinforced polyether ether ketone (CFR-PEEK).

3. A prosthesis as claimed in claim 1 or 2, wherein the surface treatment comprises a hardening treatment.

4. A prosthesis as claimed in claim 3, wherein the hardening treatment is a diffusion hardening treatment.

5. A prosthesis as claimed in any preceding claim, wherein the second component is formed from a cobalt chrome alloy.

6. A prosthesis as claimed in any preceding claim, wherein the acetabular cup is a monoblock cup.

7. A prosthesis as claimed in any preceding claim, wherein the bearing surface of the first component comprises one or more bores for receiving one or more attachment means to secure the first component in place.

## Patentansprüche

1. Prothese, umfassend:
eine erste Komponente, die eine Hüftgelenkspfanne umfasst, die aus einem faserverstärkten Polymermaterial gebildet ist und eine Auflagefläche aufweist; und
eine zweite Komponente, die eine metallische Femurkopfemeuerungskomponente umfasst, die einen kugelförmigen Kappenabschnitt und einen zentralen Stab, der sich von einer Innenfläche des kugelförmigen Kappenabschnitts erstreckt, beinhaltet, wobei die Innenfläche des kugelförmigen Kappenabschnitts und der Stab eine raue Oberfläche aufweisen, wobei die zweite Komponente ausgelegt ist, um mit der Auflagefläche der ersten Komponente zu artikulieren, wobei eine Oberflächenbehandlung auf zumindest einen Abschnitt der Auflagefläche der zweiten Komponente angewandt wird, um eine widerstandsfähige Oberfläche zu bilden, wobei die Oberflächenbehandlung eine widerstandsfähige Übergitterbeschichtung umfasst, die ein Keramikmaterial umfasst.

2. Prothese nach Anspruch 1, wobei die erste Komponente aus kohlefaserverstärktem Polyetheretherketon (CFR-PEEK) gebildet ist.

3. Prothese nach Anspruch 1 oder 2, wobei die Oberflächenbehandlung eine Härtungsbehandlung umfasst.

4. Prothese nach Anspruch 3, wobei die Härtungsbehandlung eine Diffusionshärtungsbehandlung ist.

5. Prothese nach einem vorhergehenden Anspruch, wobei die zweite Komponente aus einer Kobalt-Chrom-Legierung gebildet ist.

6. Prothese nach einem vorhergehenden Anspruch, wobei die Hüftgelenkspfanne eine Monoblockpfanne ist.

7. Prothese nach einem vorhergehenden Anspruch, wobei die Auflagefläche der ersten Komponente eine oder mehrere Bohrungen zur Aufnahme eines oder mehrerer Anbringungsmittel umfasst, um die erste Komponente zu befestigen.

## Revendications

1. Prothèse comprenant :
un premier composant comprenant une cupule acétabulaire formée à partir d'un matériau polymère renforcé de fibres et comportant une surface portante ; et
un second composant comprenant un composant de resurfaçage de tête fémorale métallique comprenant une partie calotte sphérique et une tige centrale s'étendant depuis une surface interne de la partie calotte sphérique, ladite surface interne de la partie calotte sphérique et la tige possédant une surface rugueuse, le second composant étant adapté pour s'articuler avec la surface portante du premier composant, un traitement de surface étant appliqué sur au moins une partie de la surface portante du second composant pour former une surface résistant à l'usure, le traitement de surface comprenant un revêtement en super-réseau résistant à l'usure comprenant un matériau céramique.

2. Prothèse selon la revendication 1, ledit premier composant étant formé de polyéther éther cétone renforcée de fibres de carbone (CFR-PEEK)

3. Prothèse selon la revendication 1 ou 2, ledit traitement de surface comprenant un traitement de durcissement.

4. Prothèse selon la revendication 3, ledit traitement de durcissement étant un traitement de durcissement par diffusion.

5. Prothèse selon l'une quelconque des revendications précédentes, ledit second composant étant formé à partir d'un alliage de cobalt et de chrome.

6. Prothèse selon l'une quelconque des revendications précédentes, ladite cupule acétabulaire étant une cupule monobloc.

7. Prothèse selon l'une quelconque des revendications précédentes, ladite surface portante du premier composant comprenant un ou plusieurs trous destinés à recevoir un ou plusieurs moyens de fixation pour fixer le premier composant en place.
